# DEMANDE DE BREVET EUROPEEN

(11) **EP 1 346 997 A1**
(43) Date de publication de la demande: **24.09.2003**
(21) Numéro de dépôt: 03290503.6
(22) Date de dépôt: 03.03.2003
(51) Int. Cl.: C07J 1/00, C07J 21/00

(54) **Procédé de purification de la 7alpha-hydroxy-déhydroépiandrostérone et de certains de ses dérivés, et solvates obtenus**

(30) Priorité: 22.03.2002 FR 0203629
(71) Demandeur: L'OREAL, 75008 Paris (FR)
(72) Inventeur: Fonteray, Marcel, 60840 Breuil le Sec (FR); Ly-Carry, Thi-My, 94100 St Maur (FR); Xu, Jinzhu, 75014 Paris (FR); Kompalitch, Virginie, 75006 Paris (FR)
(74) Mandataire: Dodin, Catherine

(57) **Abrégé**

L'invention se rapporte à un nouveau procédé de purification de la 7α-hydroxy-déhydroépiandrostérone ou de certains de ses dérivés, ainsi qu'aux solvates formés par la 7α-hydroxy-déhydroépiandrostérone ou un de ces dérivés, avec un alcool.

## Description

L'invention se rapporte à un nouveau procédé de purification de la 7alpha-hydroxydéhydroépiandrostérone (7α-OH-DHEA) et de dérivés de la 7α-OH-DHEA , ainsi qu'aux solvates obtenus.

Plus précisément l'invention concerne un procédé de purification d'un composé de formule (la) ou (lb): 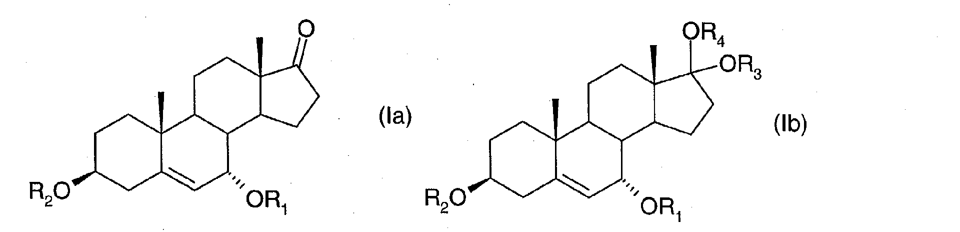 dans lesquelles :
- R₁ et R₂ désignent, indépendamment l'un de l'autre, H, alkyle, arylalkyle, alkylcarbonyle, arylcarbonyle, et
- OR₃ et OR₄ représentent, indépendamment l'un de l'autre, un groupement alkoxy comprenant 1 à 6 atomes de carbone, tels que méthoxy, éthoxy ou t-butoxy, ou OR₃ et OR₄ représentent ensemble un cycle -O-(CH₂)ₓ-O-, éventuellement substitué, dans lequel x est compris entre 1 et 6.

Le composé de formule (la) tel que R₁ et R₂ désignent H correspond à la 7alpha-hydroxydéhydroépiandrostérone (7α-OH-DHEA).

Dans l'ensemble de la description de la présente invention, le terme alkyle désigne un groupement carboné saturé, linéaire ou ramifié, contenant de 1 à 6 atomes de carbone.
Le terme alkoxy désigne un groupement RO-, dans lequel R représente un alkyle, par exemple méthoxy, éthoxy ou t-butoxy.
Le terme arylalkyle se rapporte à un radical alkyle substitué par un aryle. Le terme aryle se rapporte à un cycle ou à un bicycle aromatique tel que par exemple phényle, naphtyle, indanyle, éventuellement substitué par un à trois substituants tels que alkyle, halogène ou alkoxy.
Le terme alkylcarbonyle se réfère à un groupement R-CO-, dans lequel R représente un alkyle; on peut notamment citer un acétyle.
Le terme arylcarbonyle désigne un radical Ar-CO-, tel que Ar est une aryle.
L'expression éventuellement substitué signifie que le radical -O-(CH₂)ₓ-O- peut être substitué ou non substitué par un ou plusieurs groupes tels que halogène, alkyle, alkoxy ou hydroxy.

L'objet de l'invention réside également dans la découverte de nouveaux composés formés par le dérivé de formule (la) ou (lb) et un alcool.

La 7α-OH-DHEA est un dérivé bien décrit dans la littérature scientifique. Les différents procédés de synthèse décrits dans l'art antérieur conduisent généralement à un produit brut sous la forme d'un mélange composé de 7α-OH-DHEA, de l'isomère 7β-OH-DHEA et d'autres sous-produits.
L'obtention de la forme stéréoisomérique pure en 7α est mentionnée notamment dans les demandes FR-A1-2771105, WO-A1-92/03925 et FR-A1-2793491.
Le document FR-A1-2771105 décrit un procédé pour la préparation de la 7α-OH-DHEA à partir de la DHEA en utilisant un champignon : *Fusarium moniliforme.* Le produit formé est purifié par une chromatographie sur une colonne de gel de silice montée dans l'acétate d'éthyle et éluée par l'acétate d'éthyle. Ce procédé est coûteux et difficilement reproductible pour obtenir une grosse quantité de composé.
Le document WO-A1-92/03925 décrit un procédé de synthèse par voie chimique à partir de la 3-O-acétyl-DHEA via le dérivé 3-O-acétyl-7-bromo-DHEA. Le produit désiré est purifié par un procédé qui consiste en une dissolution du produit brut dans l'acétone ou dans le méthanol, suivie d'une précipitation avec l'hexane. Le document n'indique pas le rendement obtenu et ne précise pas la pureté isomérique en 7α-OH-DHEA. En reproduisant ce procédé, la demanderesse a isolé un mélange des isomères 7α et 7β-OH-DHEA dont la proportion de l'isomère 7β est de 30 %.
Le document FR-A1-2793491 décrit un procédé qui consiste en une synthèse stéréosélective par oxydation allylique de la position 7 de la 3-O-acétyl-DHEA. Le produit attendu est purifié par une chromatographie sur colonne de gel de silice en éluant avec un mélange hexane/acétate d'éthyle, dans les proportions initiales 50/50, progressivement enrichi en acétate d'éthyle jusqu'à 100 % en acétate d'éthyle.
En résumé, il est très laborieux, par les procédés ci-dessus mentionnés, d'obtenir la 7α-OH-DHEA ou des dérivés de la 7α-OH-DHEA purs. Par la méthode de chromatographie sur gel de silice, les temps d'élution des isomères 7α et 7β sont très proches, rendant l'opération de purification difficile, coûteuse et difficilement transposable à l'échelle industrielle. Par la méthode de précipitation, l'isomère 7β est obtenu en mélange avec l'isomère 7α.

L'objectif de la présente invention est de fournir un procédé de purification des composés de formule (la) ou (Ib), notamment de la 7α-OH-DHEA, simple, efficace, et transposable industriellement.

Le procédé de purification des composés de formule (la) ou (lb) selon l'invention consiste à recristalliser un produit brut de réaction comprenant un mélange de l'isomère 7α-OH de formule (la) ou (lb) et de l'isomère 7β-OH correspondant, et éventuellement d'autres composés, dans un solvant alcoolique ou un mélange de solvants contenant un solvant alcoolique et au moins un solvant miscible avec ledit solvant alcoolique.

Le solvant miscible avec ledit solvant alcoolique peut être choisi parmi les hydrocarbures, par exemple l'heptane, l'éther de pétrole et/ou le toluène.

On entend au sens de la présente invention par « isomère 7α-OH », le composé de formule (la) ou (lb), notamment la 7α-OH-DHEA ou le dérivé de la 7α-OH-DHEA. On entend en outre par « isomère 7β-OH », l'isomère β correspondant à l'isomère 7α-OH de formule (la) ou (lb).

Le procédé de l'invention permet avantageusement d'obtenir une pureté isomérique en isomère 7α-OH supérieure à au moins 90 %, de préférence supérieure à 95%, de préférence encore supérieure à 97%. Il met en oeuvre des solvants non toxiques d'un prix de revient modéré. Enfin, il est simple de manipulation.

Plus précisément, un premier objet de l'invention est un procédé de purification d'un dérivé 7α-OH de formule (la) ou (lb), à partir d'un mélange comprenant l'isomère 7α-OH de formule (la) ou (lb) et l'isomère 7β-OH correspondant, et éventuellement d'autres composés, ledit procédé comprenant les étapes suivantes :
- dissoudre le mélange comprenant lesdits isomères 7α-OH et 7β-OH dans un volume approprié d'un solvant alcoolique comprenant un à dix atomes de carbone, ou un mélange de solvants contenant un solvant alcoolique comprenant un à dix atomes de carbone et au moins un solvant miscible dans ledit solvant alcoolique,
- faire cristalliser l'isomère 7α-OH, et
- filtrer et laver l'isomère 7α-OH.

Selon un mode de mise en oeuvre de l'invention les composés de formule (la) avec R₁ et R₂ désignant H (7α-OH-DHEA) ou avec R₁ représentant H et R₂ représentant acétyle (3-O-acétyl-7α-OH-DHEA), sont préférés.

Le solvant alcoolique comprend de préférence 1 à 6, de préférence encore 2 à 4 atomes de carbone. Il est avantageusement choisi parmi l'éthanol, le n-propanol, le tert-butanol, l'isopropanol, l'isobutanol et le n-butanol, et leurs mélanges. On choisit de préférence l'isopropanol.

Le mélange contenant l'isomère 7α-OH et l'isomère 7β-OH est dissout dans un volume approprié dudit solvant alcoolique ou dudit mélange de solvants, de préférence 1 à 4 ml/g, de préférence encore entre 1 et 2 ml/g, de préférence encore environ égale à 2 ml/g de mélange d'isomères 7α-OH et 7β-OH.

La recristallisation peut être opérée par abaissement de la température ou par simple évaporation du solvant.

De façon avantageuse, le produit brut de réaction est dissout à chaud dans ledit solvant alcoolique ou ledit mélange de solvants, de façon préférée à reflux. Dans ce cas, le solvate de l'isomère 7α-OH cristallise avantageusement à froid, notamment par refroidissement de la solution à une température inférieure ou égale à 10°C environ.

Le solvant alcoolique ou le mélange de solvants est éventuellement éliminé par séchage sous vide.

Le procédé de l'invention est avantageusement mis en oeuvre avec un produit brut de réaction constitué d'un mélange initial d'isomère 7α-OH de formule (la) ou (lb) et d'isomère 7β-OH correspondant, comprenant au moins 70% en poids de l'isomère 7α-OH, étant entendu que le procédé de l'invention peut être mis en oeuvre avec un mélange 7α/7β en toutes proportions.

Ainsi, partant d'un brut d'un ratio 7α/7β de 95/5, on obtient généralement un produit purifié dans un ratio 7α/7β supérieur à 99/1. Partant d'un brut d'un ratio de 70/30, le ratio après purification est généralement de 98/2.

Selon un mode de mise en oeuvre de l'invention, le produit brut de réaction est obtenu par l'un des procédés décrits dans les demandes de brevet FR-A1-2 771 105, WO-A1-92/03925 et WO-A1-94/03176 incorporées dans la présente demande par référence.
Le document FR-A1-2 771 105 décrit un procédé pour la préparation de la 7α-OH-DHEA en une étape à partir de la déhydroépiandrostérone (DHEA) en utilisant le champignon *Fusarium moniliforme*.
Les documents WO-A1-94/03176 et WO-A1-92/03925 décrivent un procédé par voie chimique en quatre étapes, pour la préparation de la 7α-OH-DHEA à partir de la 3-O-acétyl-DHEA. La première étape de ce procédé permet l'obtention de la 3-O-acétyl-7-Bromo-DHEA à partir de la 3-O-acétyl-DHEA par traitement avec un agent bromant. Dans une seconde étape, le mélange racémique d'isomères 7α-Bromo sous forme majoritaire. Après équilibration, la 3-O-acétyl-7-Bromo-DHEA traitée avec un mélange d'acide acétique glacial et d'acétate d'argent permet l'obtention du 3-O-acétyl-7α-O-acétyl-DHEA. Le 3-O-acétyl-7α-O-acétyl-DHEA est ensuite traité par du carbonate de sodium (Na₂CO₃) dans un mélange constitué d'eau et de méthanol.

Selon un autre mode de mise en oeuvre de l'invention, le produit brut de réaction est obtenu selon le procédé décrit dans la demande de brevet FR2820745, incorporée dans la présente demande par référence.
Ce procédé consiste essentiellement à (i) réaliser une réaction d'oxydation en position allylique de la 3-O-acétyl-DHEA, puis à (ii) réduire la 3-O-acétyl-7-oxo-DHEA de manière régiosélective et diastéréosélective en utilisant le L-Selectride® comme agent réducteur, (iii) à déprotéger par trans-estérification l'acétate de 7α-hydroxy-DHEA pour obtenir la 7α-OH-DHEA.

Un autre objet de la présente invention est un solvate formé par la 7α-OH-DHEA ou un dérivé de la 7α-OH-DHEA et un alcool, ledit solvate ayant l'une des formules suivantes : 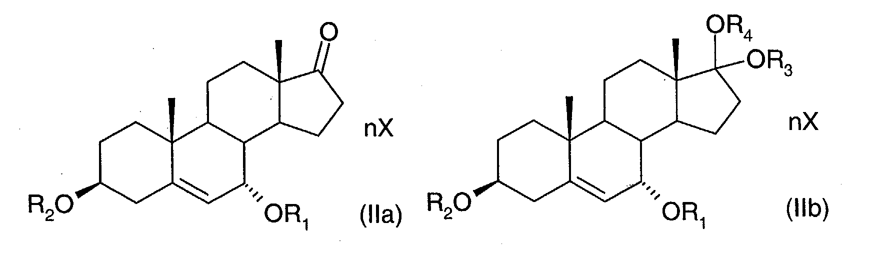 dans lesquelles
- R₁ et R₂ désignent, indépendamment l'un de l'autre, H, alkyle, arylalkyle, alkylcarbonyle, arylcarbonyle,
- OR₃ et OR₄ représentent, indépendamment l'un de l'autre, un groupement alkoxy comprenant 1 à 6 atomes de carbone, tels que méthoxy, éthoxy ou t-butoxy, ou OR₃ et OR₄ représentent ensemble un cycle -O-(CH₂)ₓ-O-, éventuellement substitué, tel que x est compris entre 1 et 6,
- n est une valeur supérieure à 0 et inférieure à 9, et
- X représente un alcool comprenant un à dix atomes de carbone, de préférence entre un et six, de préférence encore deux et quatre atomes de carbone.

Un solvate particulièrement préféré est celui pour lequel n vaut 1, et X représente un alcool aliphatique tel que l'isopropanol, le tert-butanol, l'éthanol, et/ou le n-butanol.
Un solvate particulièrement préféré est celui de formule (IIa).
Un solvate particulièrement préféré est celui pour lequel R₁ et R₂ désignent H (solvate de la 7α-OH-DHEA) ou R₁ représente H et R₂ représente acétyle (solvate de la 3-O-acétyl-7α-OH-DHEA).

Les caractéristiques physico-chimiques du solvate de 7α-OH-DHEA tel que n vaut 1 et X représente l'isopropanol sont données dans l'exemple ci-après.
La valeur de n peut être égale à 0,5, 1, 1,5, 2 ou 2,5 par exemple, car des molécules de solvant alcoolique peuvent engager des liaisons avec deux molécules d'isomère 7α-OH différentes.

### Exemple 1 : obtention du solvate 7α-OH-DHEA, isopropanol

On reproduit le procédé décrit dans l'exemple 1 de la demande de brevet FR2820745, en partant de 30g de 3-O-acétyl-7-céto-DHEA, pour synthétiser le mélange d'isomères de 7α-OH-DHEA et de 7β-OH-DHEA. Le produit brut ainsi obtenu est dissous dans 60 ml d'isopropanol (soit 2 ml/g) au reflux. La solution est ensuite refroidie à +10°C. Les cristaux sont filtrés et lavés avec un mélange d'heptane/isopropanol (1/1) froid pour donner 14,6 g d'une poudre blanche.

Caractérisations :
- RMN ¹H (DMSO-d6, 200MHz) δ (ppm) : conforme au produit attendu
- Ratio 7α/7β par HPLC : > 99 %.
- Profil DSC : pic de départ de solvant à 116,0°C et puis pic de fusion à 170,6°C, correspondant au solvate isopropanol, 7α-OH-DHEA.
- Dosage d'isopropanol par la chromatographie gazeuse : ∼ 16 %.

| Analyse élémentaire pour C₁₉H₂₈0₃.C₃H₈0 : | | | |
|---|---|---|---|
| Théorie % | C : 72,48 | H : 9.95 | O : 17,56 |
| Trouvé % | C : 72,88 | H : 9.88 | O : 16,76 |

### Exemple 2

L'exemple 1 est reproduit en utilisant d'autres solvants alcooliques. Les résultats obtenus sont regroupés dans le tableau suivant :

| Solvant | quantité de solvant de recristallisation | Ratio 7α/7β par HPLC |
|---|---|---|
| éthanol | 1 ml/g | 99,2/0,8 |
| n-butanol | 1 ml/g | 98,4/1,6 |
| tert-butanol | 2,3 ml/g | 99,5/0,5 |

## Revendications

1. Solvate de la 7-alpha-hydroxy-déhydroépiandrostérone (7α-OH-DHEA) ou d'un dérivé de la 7α-OH-DHEA avec un alcool, ledit solvate ayant l'une des formules suivantes : 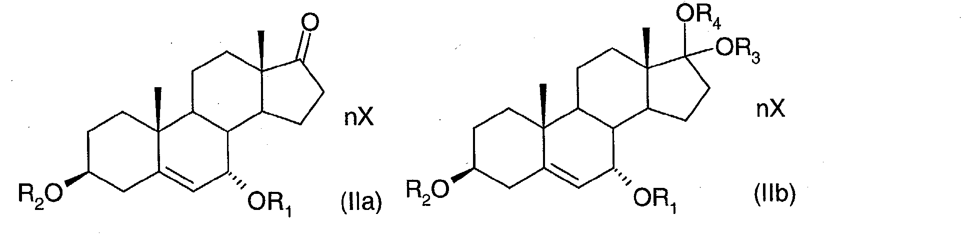 dans lesquelles
- R₁ et R₂ désignent, indépendamment l'un de l'autre, H, alkyle, arylalkyle, alkylcarbonyle, arylcarbonyle,
- OR₃ et OR₄ représentent, indépendamment l'un de l'autre, un groupement alkoxy comprenant 1 à 6 atomes de carbone, tels que méthoxy, éthoxy ou t-butoxy, ou OR₃ et OR₄ représentent ensemble un cycle -O-(CH₂)ₓ-O-, éventuellement substitué, tel que x est compris entre 1 et 6,
- n est une valeur supérieure à 0 et inférieure à 9, et
- X représente un alcool comprenant un à dix atomes de carbone, de préférence entre un et six, de préférence encore deux et quatre atomes de carbone.

2. Solvate selon la revendication 1, **caractérisé en ce qu'**il est de formule (IIa) avec soit R₁ et R₂ représentant H, soit R₁ représentant H et R₂ représentant acétyle.

3. Solvate selon l'une des revendications précédentes, **caractérisé en ce que** X représente un alcool aliphatique tel que l'isopropanol, le tert-butanol, l'éthanol, et/ou le n-butanol.

4. Solvate selon l'une des revendications précédentes, **caractérisé en ce que** X représente l'isopropanol et n vaut 1.

5. Procédé de purification d'un composé de formule (la) ou (lb): 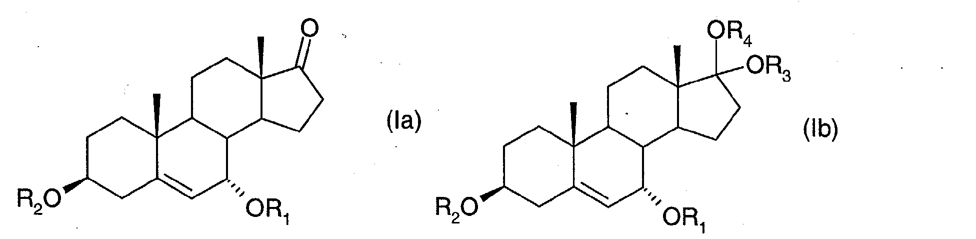 dans lesquelles :
- R₁ et R₂ désignent, indépendamment l'un de l'autre, H, alkyle, arylalkyle, alkylcarbonyle, arylcarbonyle, et
- OR₃ et OR₄ représentent, indépendamment l'un de l'autre, un groupement alkoxy comprenant 1 à 6 atomes de carbone, tels que méthoxy, éthoxy ou t-butoxy, ou OR₃ et OR₄ représentent ensemble un cycle -O-(CH₂)ₓ-O-, éventuellement substitué, dans lequel x est compris entre 1 et 6,
à partir d'un mélange comprenant l'isomère 7α-OH de formule (la) ou (lb) et l'isomère 7β-OH correspondant, ledit procédé comprenant les étapes consistant à :
- dissoudre le mélange comprenant lesdits isomères 7α-OH et 7β-OH dans un volume approprié d'un solvant alcoolique comprenant un à dix atomes de carbone, ou un mélange de solvants contenant un solvant alcoolique comprenant un à dix atomes de carbone et au moins un solvant miscible dans ledit solvant alcoolique,
- faire cristalliser l'isomère 7α-OH, et
- filtrer et laver l'isomère 7α-OH.

6. Procédé selon la revendication 5, **caractérisé en ce que** le composé est de formule (la) avec R₁ et R₂ désignant H (7α-OH-DHEA) ou R₁ représentant H et R₂ représentant acétyle.

7. Procédé selon l'une des revendications 5 à 6, **caractérisé en ce que** le solvant alcoolique comprend 1 à 6, de préférence 2 à 4 atomes de carbone, et est choisi parmi l'isopropanol, l'éthanol, le n-propanol, le tert-butanol, l'isobutanol, le n-butanol et leurs mélanges.

8. Procédé selon l'une des revendications 5 à 7, **caractérisé en ce que** le volume approprié de solvant alcoolique ou dudit mélange de solvants est compris entre 1 à 4 ml/g, de préférence compris entre 1 et 2 ml/g, de préférence encore environ égal à 2 ml par gramme de mélange d'isomère 7α-OH et d'isomère 7β-OH.

9. Procédé selon l'une des revendications 5 à 8, **caractérisé en ce que** le mélange d'isomère 7α-OH et d'isomère 7β-OH est dissout à chaud dans le solvant alcoolique ou le mélange de solvants, en particulier à reflux.

10. Procédé selon l'une des revendications 5 à 9, **caractérisé en ce que** le mélange initial d'isomère 7α-OH et d'isomère 7β-OH comprend au moins 70% en poids d'isomère 7α-OH.
